# EUROPÄISCHE PATENTSCHRIFT

(11) **EP 3 019 102 B1**
(45) Veröffentlichungstag und Bekanntmachung des Hinweises auf die Patenterteilung: **31.08.2022**
(21) Anmeldenummer: 14737220.5
(22) Anmeldetag: 07.07.2014
(51) Int. Cl.: A61B 17/86, A61C 8/00

(54) **KNOCHENSCHRAUBE**
BONE SCREW
VIS D'OSTÉOSYNTHÈSE

(30) Priorität: 08.07.2013 DE 102013107170
(43) Veröffentlichungstag der Anmeldung: 18.05.2016
(73) Patentinhaber: Aesculap AG, 78532 Tuttlingen (DE)
(72) Erfinder: PEUKERT, Andrea, 78532 Tuttlingen (DE); KRÜGER, Sven, 78647 Trossingen (DE); RAUSCHMANN, Michael, Prof. Dr. med., 60439 Frankfurt am Main (DE); BOSZCZYK, Bronek, Dr. med., Wollaton Nottingham NG8 2NQ (GB)
(74) Vertreter: Winter, Brandl - Partnerschaft mbB
(86) Internationale Anmeldenummer: PCT/EP2014/064464
(87) Internationale Veröffentlichungsnummer: WO 2015/004063

(56) Entgegenhaltungen:
- WO-A1-00/32125
- WO-A1-2012/103354
- DE-U1-202012 009 023
- US-A- 3 466 748
- US-A1- 2012 215 263
- US-A1- 2012 232 600

## Beschreibung

Die vorliegende Erfindung betrifft eine Pedikelschraube, gemäß dem Oberbegriff des Anspruchs 1.

Pedikelschrauben dienen prinzipiell zur dorsalen Stabilisierung der Wirbelsäule bei Frakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger oder Steg geschaffen wird. Die Pedikelschrauben und Längsträger bilden dabei ein Wirbel-Stabilisierungssystem.

Die Pedikelschrauben werden zwischen dem Querfortsatz (Processus transversus) und dem Dornfortsatz (Processus spinosus) durch den Pedikel in den Wirbelkörper (Corpus vertebrae) geschraubt. Hierfür hat eine Pedikelschraube in der Regel einen Schraubenkopf und einen mit einem Außengewinde versehenen länglichen Schraubenschaft. Während der Schraubenkopf zur Fixierung des Längsträgers tulpenförmig ausgebildet ist oder eine sogenannte Tulpe trägt, dient der Schraubenschaft zur Verankerung der Pedikelschraube im Wirbel.

Hinsichtlich der Gestaltung des Schraubenschafts sind unterschiedliche Geometrien bekannt.

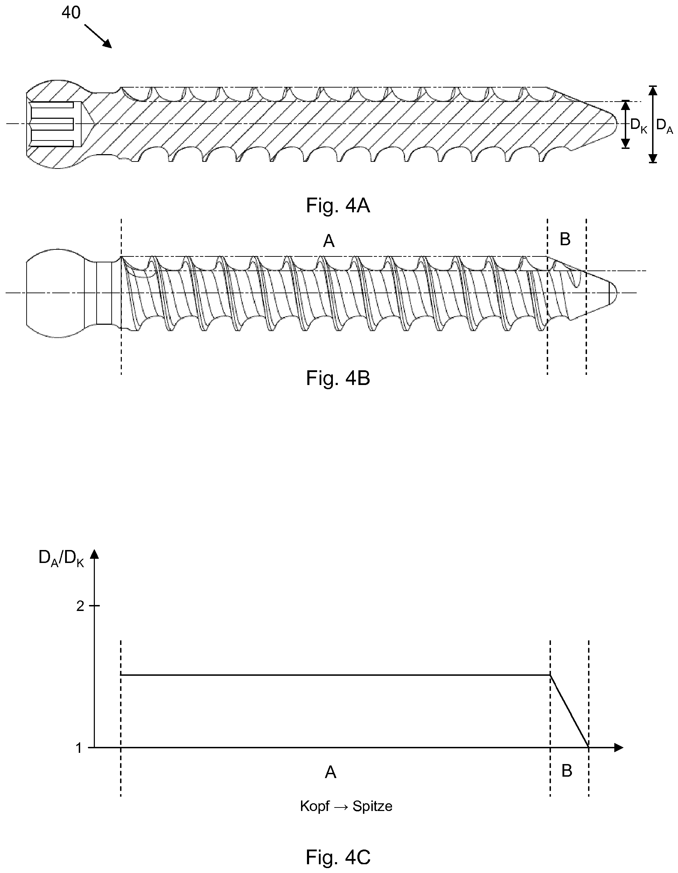

Beispielsweise werden Schrauben mit einem zylindrischen Gewinde verwendet, bei denen sowohl der Kerndurchmesser als auch der Außen- bzw. Flankendurchmesser über die gesamte Längserstreckung des Schraubenschafts konstant bleiben (vgl. Fig. 4A). Diese weisen eine sehr hohe Ausreißfestigkeit auf, die selbst bei einem Zurückdrehen der Schraube, um zum Beispiel nachträglich die Position des Schraubenkopfs zu korrigieren, kaum beeinträchtigt wird. Nachteilig an einem gleichbleibenden Außendurchmesser und Kerndurchmesser ist, dass das Gewinde, das durch das Außengewinde in dem Eintrittsbereich zwischen Querfortsatz und Dornfortsatz in den Knochen geschnitten wird, beim weiteren Eindrehen der Schraube ausleiern kann, wodurch der Halt im Bereich des proximalen Schaftabschnitts verringert sein kann. Ein weiterer Nachteil ist darin zu sehen, dass ein zylindrisches Gewinde, insbesondere ein gleichbleibender Kerndurchmesser, nicht in der Lage ist, das im Wirbel vorhandene spongiöse Knochenmaterial zwischen den Gewindeflanken zu verdichten.

Ferner sind Schrauben mit einem vollkonischen Gewinde bekannt, bei welchen sowohl der Gewindeaußendurchmesser als auch der Kerndurchmesser von der Schraubenspitze bis zum Schraubenkopf gleichmäßig zunehmen. Der Vorteil dieser Schraubengeometrie ist darin zu sehen, dass diese gut im Knochen greifen und auch noch der proximale Schaftabschnitt weiter in den Knochen schneidet und dadurch auch im proximalen Bereich ein guter Halt gewährleistet ist. Schrauben mit einem vollkonischen Gewinde erlauben jedoch kein nachträgliches Feinjustieren der Schrauben, da ansonsten die Gewindeflanken der Schraube selbst bei nur geringem Zurückdrehen über die gesamte Länge den Kontakt mit dem zuvor in radialer Richtung zu sehr aufgeweiteten Knochengewinde verlieren.

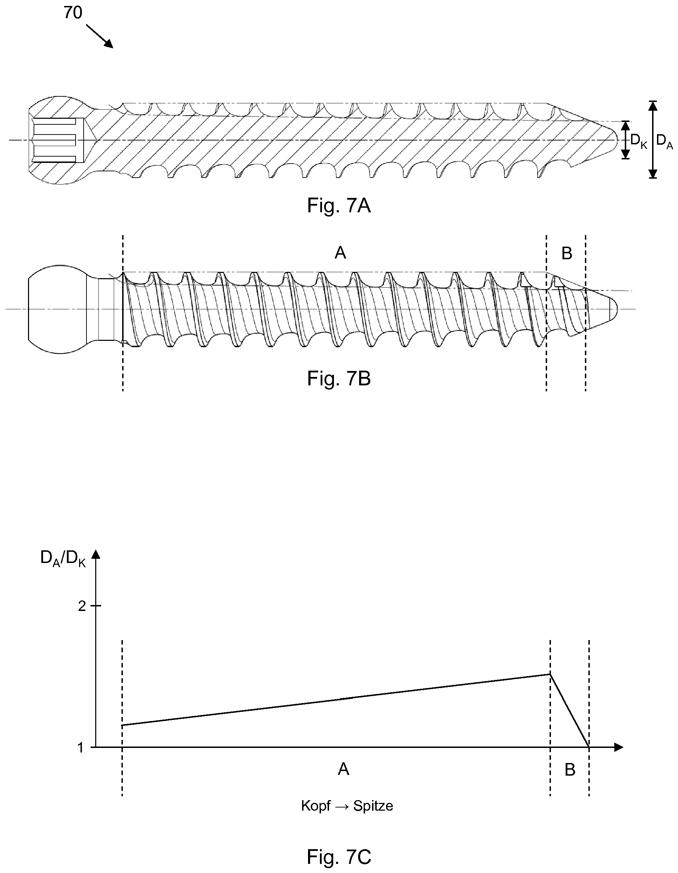

Aus diesem Grund werden oft auch Schrauben mit einem teilkonischen Gewinde verwendet, bei denen der Kern konisch und das Gewinde zylindrisch ausgebildet sind (vgl. Fig. 7A). Das zylindrische Gewinde gewährt eine hohe Ausreißfestigkeit. Durch den zunehmend größer werdenden Kerndurchmesser wird zwischen den Gewindeflanken radialer Druck auf das spongiöse Knochenmaterial erzeugt und dieses verdichtet, wodurch der Halt verbessert wird. Darüber hinaus weist eine Schraube mit teilkonischem Gewinde aufgrund des größer werdenden Kerndurchmessers auch eine höhere Dauerfestigkeit als eine Schraube mit einem konstanten Kerndurchmesser auf.

Bei Schrauben mit teilkonischem Gewinde werden jedoch die Gewindeflanken zum Schraubenkopf hin immer breiter und stumpfer, weshalb diese im proximalen Bereich des Schraubenschafts nicht mehr schneiden, sondern radial und axial in den Gewindegang drücken und gegebenenfalls den Gewindegang im proximalen Bereich aufweiten oder sogar sprengen. Auch in diesem Fall kann ein leichtes Zurückdrehen zu einer Lockerung der Schraube führen, da die Gewindeflanken den Kontakt mit dem (in axialer Richtung) aufgeweiteten Gewindegang verlieren.

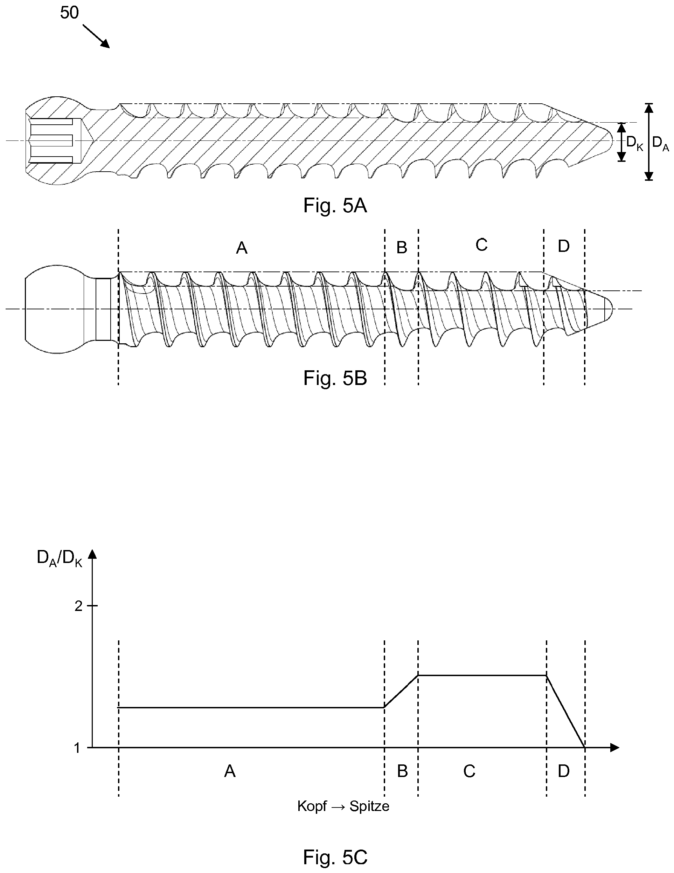

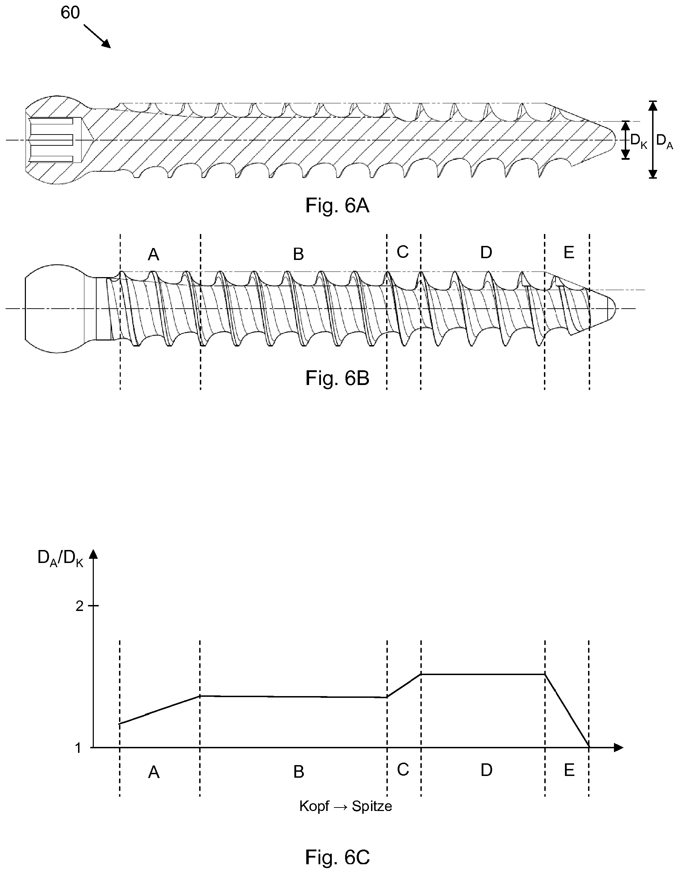

Da jede dieser Geometrien Vor- und Nachteile aufweist, sind auch Kombinationen aus diesen Geometrien bekannt, wie z.B. eine Schraube mit einem zylindrischen Außendurchmesser und einen Kern mit abwechselnden zylindrischen und konischen Kerndurchmesserabschnitten (vgl. Fign. 5A und 6A).

DE 20 2012 009023 U1 und WO 00/32125 A1 offenbaren Pedikelschrauben, bei welchen jeweils der Gewindeaußendurchmesser und der Gewindekerndurchmesser in Richtung des entsprechenden Schraubenkopfs zunehmen, wobei die Differenz zwischen Gewindeaußendurchmesser und Gewindekerndurchmesser gleich bleibt. Das heißt, dass sich die Gewindetiefe in Richtung des entsprechenden Schraubenkopfs nicht vergrößert.

WO 2012/103354 A1 offenbart gattungsfremde Schrauben zur Heilung von Armbrüchen und US 3,466,748 A offenbart gattungsfremde Schrauben für Zahnprothesen.

Wie oben bereits erwähnt befindet sich die Eintrittsstelle einer Pedikelschraube zwischen dem Querfortsatz und dem Dornfortsatz, verläuft dann in axialer Richtung des relativ schmalen Pedikels durch den Pedikel und mündet im Wirbelkörper. Im Bereich des Wirbelkörpers und im Bereich zwischen Eintrittsstelle und Pedikel liegt überwiegend weiches spongiöses Knochenmaterial vor, wohingegen im dünnen Bereich des Pedikels und unmittelbar im Bereich der Eintrittsstelle dichtes kortikales Knochenmaterial anzutreffen ist. Keine der bislang bekannten Gewindeformen wird diesem Knochenaufbau eines Wirbels und der inhomogenen Knochenfestigkeit gerecht.

Aufgabe der vorliegenden Erfindung ist somit die Bereitstellung einer Pedikelschraube, die einen optimalen Halt gewährleistet, wenn sie in einem Knochen mit kortikaler Knochenhülle und spongiösem Knochenkern verankert wird.

Diese Aufgabe wird durch die Merkmale des Anspruchs 1 gelöst. Vorteilhafte Weiterbildungen sind Gegenstand der Unteransprüche.

Eine erfindungsgemäße Pedikelschraube, weist einen Schraubenschaft und einen am proximalen Ende des Schraubenschafts vorgesehenen Schraubenkopf auf. Der Schraubenschaft ist zumindest abschnittsweise mit einem ein- oder mehrgängigen, selbstschneidenden Außengewinde versehen, um die Knochenschraube im Knochen zu verankern. Dabei nimmt das Verhältnis von Gewindeaußendurchmesser zu Gewindekerndurchmesser in einem proximalen Schraubenschaftabschnitt oder im Bereich des Gewindeauslaufs zum Schraubenkopf hin zu.

Dies bedeutet, dass die Gewindegänge im Bereich des Gewindeauslaufs tiefer werden und sich noch tiefer in das Knochenmaterial einschneiden. Das Gewinde wird zum Schraubenkopf hin noch "aggressiver" und weist insbesondere selbst bei der letzten Umdrehung ein sehr gutes Greifverhalten auf.

Insbesondere der proximale Gewindeabschnitt bzw. der Gewindeauslauf befindet sich im Bereich der Eintrittstelle der Schraube in den Knochen, d.h. im Bereich der dichten kortikalen Hülle. Indem sich die scharfen Gewindeflanken immer tiefer in dieses kortikale Knochenmaterial hinein schneiden, wird eine verbesserte Verankerung der Schraube im Knochen ermöglicht.

Die erfindungsgemäße Gestaltung des Gewindeauslaufs lässt sich unter anderem auf eine Pedikelschraube anwenden, da der proximale Schraubenschaftabschnitt einer Pedikelschraube - im Gegensatz zum medialen und distalen Schraubenschaftabschnitts - nicht durch die Abmessungen des recht dünnen Pedikels beschränkt wird. Im Bereich zwischen Eintrittsstelle und Pedikel ist mehr Raum, so dass in diesem Bereich wie im Anspruch 1 vorgeschlagen das Verhältnis von Gewindeaußendurchmesser zu Gewindekerndurchmesser erhöht werden kann, um die oben genannten Vorteile zu erzielen.

Erfindungsgemäß nimmt das Verhältnis von Gewindedurchmesser zu Gewindekerndurchmesser in einem Übergangsbereich von einem medialen Schraubenschaftabschnitt zu dem proximalen Schraubenabschnitt zunächst ab, bevor es in dem proximalen Schraubenschaftabschnitt zunimmt.

Gemäß einer Weiterbildung der Erfindung nimmt in dem proximalen Schraubenschaftabschnitt bzw. im Bereich des Gewindeauslaufs sowohl der Gewindeaußendurchmesser als auch der Gewindekerndurchmesser zu, wobei der Gewindeaußendurchmesser verhältnismäßig stärker zunimmt.

Die Zunahme des Kerndurchmesser führt dazu, dass die Schraube auf das zwischen den Gewindeflanken befindliche und im Bereich zwischen Eintrittsstelle und Pedikel überwiegend spongiöse Knochenmaterial einen radialen Druck ausübt und verdichtet, so dass die Schraube nicht nur über die Gewindeflanken (formschlüssig), sondern auch durch diesen radialen Druck (kraftschlüssig) besser im Knochenmaterial fixiert wird.

Durch den größeren Kerndurchmesser wird auch die Dauerfestigkeit der Schraube erhöht.

Dadurch, dass das Verhältnis von Gewindeaußendurchmesser zu Gewindekerndurchmesser zum Schraubenkopf hin zunimmt, wird sichergestellt, dass trotz zunehmenden Kerndurchmessers auch die letzten Gewindeumdrehungen scharf und schneidend bleiben und nicht wie bei dem oben im Stand der Technik beschriebenen teilkonischen Gewinde breit und stumpf.

Somit verbindet die erfindungsgemäße Geometrie die Vorteile eines zylindrischen Gewindeauslaufs mit den Vorteilen eines teilkonischen Gewindeauslaufs, d.h. einerseits schneiden sich die bis zum letzten Gewindegang scharfen Gewindeflanken in die kortikale Knochenhülle und sorgen für eine verbesserte Verankerung und andererseits wird das (spongiöse) Knochenmaterial beim Eindrehen der Schraube durch den zunehmenden Kerndurchmesser verdichtet.

Gemäß einer Weiterbildung der Erfindung ist in dem proximalen Schraubenschaftabschnitt der Gewindekerndurchmesser zylindrisch bzw. konstant, während der Gewindeaußendurchmesser in diesem Abschnitt zunimmt, weshalb das Verhältnis von Gewindeaußendurchmesser zu Gewindekerndurchmesser zum Schraubenkopf hin zunimmt.

Gemäß einer Weiterbildung der Erfindung nehmen in dem proximalen Schraubenschaftabschnitt sowohl der Gewindeaußendurchmesser als auch der Gewindekerndurchmesser linear zu. Mit anderen Worten sowohl der Gewindeaußendurchmesser als auch der Gewindekerndurchmesser nehmen konisch zu. Diese Geometrie ist zum einen leichter zu fertigen, zum anderen lässt sich der proximale Gewindeabschnitt mit gleich bleibendem Drehmoment in den Knochen drehen.

Gemäß einer Weiterbildung der Erfindung nehmen in dem proximalen Schraubenschaftabschnitt sowohl der Gewindeaußendurchmesser als auch der Gewindekerndurchmesser exponentiell zu. Mit anderen Worten nehmen der Gewindeaußendurchmesser als auch der Gewindekerndurchmesser zum Schraubenkopf hin immer stärker zu, so dass gerade am proximalen Gewindeende, das im Bereich der Eintrittsstelle liegt, besonders tief in das dichte kortikalen Knochenmaterial schneidet und für eine besonders gute Verankerung sorgt.

Gemäß einer Weiterbildung der Erfindung nehmen in dem proximalen Schraubenschaftabschnitt der Gewindeaußendurchmesser exponentiell und der Gewindekerndurchmesser linear zu. Mit anderen Worten nimmt der Gewindeaußendurchmesser insbesondere am proximalen Gewindeende, das im Bereich der Eintrittsstelle liegt, im Vergleich zum Kerndurchmesser wesentlich stärker zu, wodurch die Gewindetiefe stark zunimmt. Dadurch wird eine gute Verankerung in der kortikalen Knochenhülle ermöglicht.

Gemäß einer Weiterbildung der Erfindung bleiben in einem medialen Schraubenschaftabschnitt der Gewindeaußendurchmesser und der Gewindekerndurchmesser konstant. Diese Geometrie wird insbesondere den Anforderungen einer Pedikelschraube gerecht, da im Bereich des Pedikels der Knochenquerschnitt nicht sehr groß ist und keine großen Variationen im Schraubendurchmesser zulässt.

Gemäß einer Weiterbildung der Erfindung nimmt in einem Übergangsbereich vom medialen Schraubenschaftabschnitt zum proximalen Schraubenschaftabschnitt hin der Gewindekerndurchmesser zu, während der Gewindeaußendurchmesser konstant bleibt. Mit anderen Worten nimmt das Verhältnis Gewindeaußendurchmesser zu Gewindekerndurchmesser in dem Übergangsbereich zunächst ab, bevor es im proximalen Schraubenschaftabschnitt zunimmt. Da der Übergangsbereich im Wesentlichen im Bereich zwischen Eintrittsstelle und Pedikel liegt, in dem überwiegend weiches spongiöses und weniger kortikales Knochenmaterial vorzufinden ist, kann durch die Erhöhung des Kerndurchmessers radialer Druck auf das spongiöse Knochenmaterial ausgeübt und dieses verdichtet werden, ohne dabei den Schraubendurchmesser insgesamt zu erhöhen. Die Erhöhung des Gewindeaußendurchmessers bzw. des Durchmesserverhältnis kann somit auf die letzten Gewindeumdrehungen, die im Bereich der kortikalen Knochenhülle liegen, beschränkt werden, da die scharfen Gewindeflanken insbesondere für das dichte kortikale Knochenmaterial notwendig sind.

Gemäß einer Weiterbildung der Erfindung nimmt in einem distalen Schraubenschaftabschnitt der Gewindeaußendurchmesser und der Gewindekerndurchmesser zur Schraubenspitze hin ab. Da das Gewinde bis in die Schraubenspitze reicht, kann die Schraube ohne große Vorbohrung und ohne großen Druck in das Knochenmaterial eingebracht werden.

Gemäß einer Weiterbildung der Erfindung sind der Schraubenkopf und der Schraubenschaft separat ausgebildet und miteinander koppelbar. Dies vereinfacht die Fertigung der jeweiligen Einzelteile und erhöht die Flexibilität und Anpassbarkeit der jeweiligen Komponenten.

Gemäß einer Weiterbildung der Erfindung ist mit dem Schraubenkopf eine Tulpe zur Fixierung eines Längsträgers eines Wirbel-Stabilisierungssystems derart koppelbar, dass die Tulpe bezüglich der Schraubenlängsachse schwenk- und/oder drehbar ist. Somit kann die erfindungsgemäße Knochenschraube auch Teil einer polyaxialen Pedikelschraube sein.

### Kurze Beschreibung der Zeichnungen

Die Erfindung wird anhand mehrerer Ausführungsformen und Vergleichsbeispielen unter Bezugnahme auf die beigefügten Zeichnungen beschrieben.
Fign. 1A und 1B zeigen eine Pedikelschraube gemäß einer ersten Ausführungsform der Erfindung in einer Querschnittsansicht und einer Seitenansicht und Fig. 1C zeigt das Verhältnis von Gewindeaußendurchmesser zu Gewindekerndurchmesser über die Länge des Gewindes;
Fig. 2 zeigt eine Querschnittsansicht eines Wirbels mit einer Pedikelschraube gemäß der ersten Ausführungsform der Erfindung im eingeschraubten Zustand;
Fign. 3A und 3B zeigen eine Pedikelschraube gemäß einer zweiten Ausführungsform der Erfindung in einer Querschnittsansicht und einer Seitenansicht und Fig. 3C zeigt ein Verhältnis von Gewindeaußendurchmesser zu Gewindekerndurchmesser über die Gewindelänge;
Fign. 4A und 4B zeigen eine herkömmliche Pedikelschraube mit zylindrischem Gewinde in einer Querschnittsansicht und einer Seitenansicht und Fig. 4C zeigt das Verhältnis von Gewindeaußendurchmesser zu Gewindekerndurchmesser über die Gewindelänge;
Fign. 5A und 5B zeigen eine herkömmliche Pedikelschraube mit einem zylindrischen Gewinde und einem ersten zylindrischen, einem konischen und einem zweiten Gewindekerndurchmesserabschnitt und Fig. 5C zeigt das entsprechende Verhältnis von Gewindeaußendurchmesser zu Gewindekerndurchmesser über die Gewindelänge;
Fign. 6A und 6B zeigen eine herkömmliche Pedikelschraube mit einem zylindrischen Gewinde und einem ersten zylindrischen, einem ersten konischen, einem zweiten zylindrischen und einem zweiten konischen Gewindekerndurchmesserabschnitt und Fig. 6C zeigt das entsprechende Verhältnis von Gewindeaußendurchmesser zu Gewindekerndurchmesser über die Gewindelänge; und
Fign. 7A und 7B zeigen eine herkömmliche Pedikelschraube mit einem zylindrischen Gewindeaußendurchmesser und einem konischen Gewindekerndurchmesser und Fig. 7C zeigt das entsprechende Verhältnis von Gewindeaußendurchmesser zu Gewindekerndurchmesser über die Gewindelänge.

### Detaillierte Beschreibung von bevorzugten Ausführungsformen und

### Vergleichsbeispielen

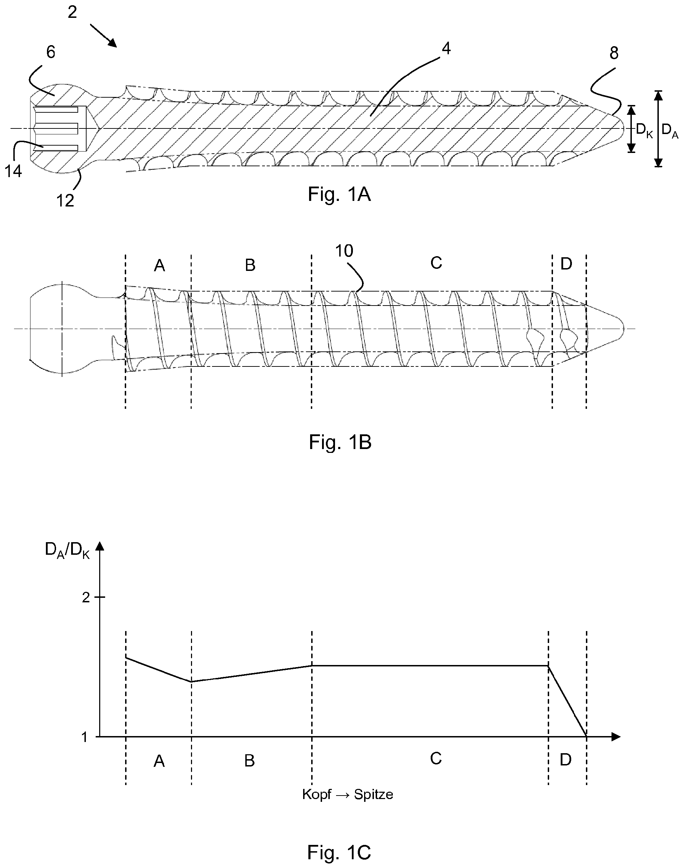

Die Fign. 1A und 1B zeigen in einer Querschnittsansicht bzw. einer Seitenansicht eine erfindungsgemäße Knochenschraube bzw. Pedikelschraube 2. Die Pedikelschraube 2 weist einen länglichen Schraubenschaft 4 auf, an dessen proximalen Ende ein Schraubenkopf 6 vorgesehen ist und der an seinem distalen Ende zu einer Schraubenspitze 8 konisch zuläuft.

Der Schraubenschaft 4 ist mit einem Außengewinde 10 versehen. Dabei handelt es sich bei der gezeigten Ausführungsform um ein selbstschneidendes, eingängiges Schraubengewinde. Alternativ kann auch ein doppel- oder mehrgängiges Gewinde vorgesehen sein.

Der Schraubenkopf 6, der einstückig mit dem Schraubenschaft 4 ausgebildet ist, weist eine sphärische Oberfläche 12 und eine axiale Ausnehmung 14 mit einem Innensechskant oder einer anderweitigen Formgebung auf, um mit einem entsprechenden Werkzeug die Pedikelschraube 2 in das Knochenmaterial eindrehen zu können. Der Schraubenkopf 6 und der Schraubenschaft 4 können jedoch auch zweiteilig ausgebildet sein.

Die Kugelkopfform des Schraubenkopfs 6 dient zur polyaxialen Ausrichtung einer so genannten Tulpe, mit welcher die Pedikelschraube 2 zusammen wirkt und welche zur Aufnahme und Fixierung eines Trägers dient, über welchen mehrere Pedikelschrauben im Rahmen eines Wirbelstabilisierungssystems verbunden werden können.

Im Folgenden wird detaillierter auf die Gestaltung des Außengewindes 10 Bezug genommen.

Das Außengewinde 10 erstreckt sich über mehrere Schraubenschaftabschnitte A bis D mit jeweils unterschiedlichen Gewindeverläufen bzw. unterschiedlichen Verhältnissen von Gewindeaußendurchmesser D_{A} zu Gewindekerndurchmesser D_{K}. Diese unterschiedlichen Schraubenschaftabschnitte A bis D sind zum einen in der Fig. 1B und zum anderen in dem in der Fig. 1C gezeigten (nicht maßstabsgetreuen) Diagramm dargestellt.

Der Schraubenschaft 4 weist einen relativ langen Schraubenschaftabschnitt C auf, in dem sich weder der Gewindeaußendurchmesser D_{A} noch der Gewindekerndurchmesser D_{K} ändern, sondern konstant bleiben. Auf der distalen Seite dieses Schaftabschnitts C mit konstantem zylindrischem Gewinde befindet sich der Schaftabschnitt D, in welchen sich der Gewindeaußendurchmesser D_{A} zur Schraubenspitze 8 hin zunächst auf den Gewindekerndurchmesser D_{K} verringert bzw. ausläuft, bevor sich der Kerndurchmesser D_{K} zur Schraubenspitze 8 hin verjüngt.

Zur Seite des Schraubenkopfs 6 hin vergrößert sich in einem Übergangsbereich B zunächst bei gleich bleibendem Gewindeaußendurchmesser D_{A} der Gewindekerndurchmesser D_{K}, wodurch das Verhältnis von Gewindeaußendurchmesser D_{A} zu Gewindekerndurchmesser D_{K} entsprechend abnimmt, bevor im proximalen Gewindeabschnitt bzw. Gewindeauslauf A das Verhältnis von Gewindeaußendurchmesser D_{A} zu Gewindekerndurchmesser D_{K} wieder ansteigt. Genauer gesagt nehmen im Bereich des Gewindeauslaufs A sowohl der Gewindeaußendurchmesser D_{A} als auch der Gewindekerndurchmesser D_{K} zu, wobei der Gewindeaußendurchmesser D_{A} stärker zunimmt.

Wenn das Verhältnis von Gewindeaußendurchmesser D_{A} zu Gewindekerndurchmesser D_{K} zunimmt, bedeutet dies, dass das Gewinde tiefer bzw. die Gewindeflanken höher werden und sich so die Pedikelschraube 2 in diesem Bereich tiefer in das Knochenmaterial einschneiden kann. Wenn dagegen das Verhältnis von Gewindeaußendurchmesser D_{A} zu Gewindekerndurchmesser D_{K} abnimmt, wie dies in den Abschnitten B und D der Fall ist, wird das Gewinde 10 insgesamt flacher und stumpfer.

In den Fign. 1A und 1B sind die vom Gewindeaußendurchmesser und Gewindekerndurchmesser beschriebenen Hüllkurven durch zweifach gepunktete Strichlinien angedeutet. Der Verlauf des Gewindeaußendurchmessers D_{A} und des Gewindekerndurchmessers D_{K} kann wie in den Fign. 1A und 1B in einer Kurve bzw. exponentiell ansteigen. Alternativ hierzu kann die Zunahme der beiden Durchmesser D_{A} bzw. D_{K} linear bzw. konisch erfolgen oder kann der eine von beiden exponentiell und der andere von beiden linear zunehmen, solange sichergestellt ist, dass zumindest im Gewindeauslaufbereich, d.h. im Schraubenschaftabschnitt A bzw. am proximalen Gewindeende das Verhältnis von Gewindeaußendurchmesser D_{A} zu Gewindekerndurchmesser D_{K} zum Schraubenkopf 6 hin tendenziell zunimmt.

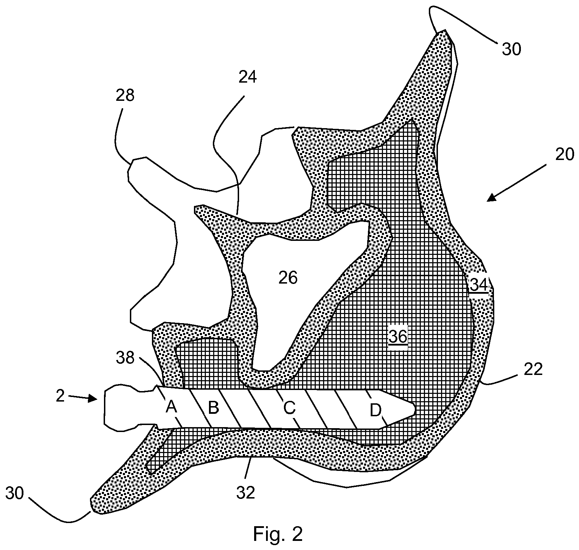

Die vorteilhafte Wirkung der erfindungsgemäßen Pedikelschraube 2 wird anhand der Fig. 2 dargestellt, welche die Pedikelschraube 2 im eingeschraubten Zustand in einem Wirbel 20 zeigt. Der Wirbel 20 weist einen Wirbelkörper 22 und einen Wirbelbogen 24, der einen Wirbelkanal 26 umschließt, auf. Der Wirbel 20 weist ferner einen Dornfortsatz 28 und zu beiden Seiten jeweils einen Querfortsatz 30 auf. Die Pedikelschraube 2 wird zwischen Dornfortsatz 28 und Querfortsatz 30 über den so genannten Pedikel 32 in den Wirbelkörper 22 eingeschraubt und so im Wirbel 20 fixiert.

Aus der Fig. 2 ist ferner erkennbar, dass der Wirbel 20 eine dichte und harte Hülle aus kortikalem Knochen 34 und im Inneren, insbesondere im Bereich des Wirbelkörpers 22 aus weichem spongiösem Knochen 36 aufgebaut ist. Die Schraubengeometrie im Bereich des medialen und distalen Schraubenschaftabschnitts wird im Wesentlichen durch die relativ dünnen Abmessungen des Pedikels 32 beschränkt. Da sich der Knochen im Bereich des Pedikels verengt, besteht der Pedikel 32 überwiegend aus kortikalem Knochenmaterial 34.

Wie in der Fig. 2 ferner erkennbar ist, durchdringt die Pedikelschraube 2 von einer Eintrittsstelle 38 bis hin zum Wirbelkörper 22 unterschiedliche Knochenschichten. Der Wirbel 22 weist im Bereich der Eintrittsstelle 38 kortikales Knochenmaterial 34, im Bereich zwischen Eintrittsstelle 38 und Pedikel 32 überwiegend spongiöses Knochenmaterial 36, im sich verengenden Bereich des Pedikels 32 überwiegend kortikales Knochenmaterial 34 und im Bereich des Wirbelkörpers 22 wieder spongiöses Knochenmaterial 36 auf.

Da die Pedikelschraube 2 im Bereich des Gewindeauslaufs bzw. Schraubenschaftabschnitts A aufgrund des dort zunehmenden Verhältnisses von Gewindeaußendurchmesser D_{A} zu Gewindekerndurchmesser D_{K} tiefe und scharfe Gewindeflanken aufweist, können sich diese sehr gut in den dort vorhandnen kortikalen Knochen 34 einschneiden und verankern.

Im Übergangsbereich B zwischen dem Gewindeauslauf A und dem mittleren Schraubenschaftabschnitt C weist die Pedikelschraube 2 einen zum Schraubenkopf 6 hin größer werdenden Kerndurchmesser D_{K} auf, so dass über den größer werdenden Kerndurchmesser D_{K} beim Eindrehen der Pedikelschraube 2 das zwischen der Eintrittsstelle 38 und dem Pedikel 32 vorliegende spongiöse Knochenmaterial 36 durch radialen Druck verdichtet wird, wodurch die Pedikelschraube 2 auch in diesem Bereich einen guten Halt in dem spongiösen Knochenmaterial 36 erzielt.

Da die Pedikelschraube 2 im medialen Schraubenschaftabschnitt C ein zylindrisches Gewinde mit gleich bleibendem Gewindeaußendurchmesser D_{A} und Gewindekerndurchmesser D_{K} aufweist, kann sich die Pedikelschraube 2 auch sehr gut in das kortikale Knochenmaterial 34 des Pedikels 32 einschneiden und dort verankern.

Somit trägt die Pedikelschraube 2 mit ihren unterschiedlichen Abschnitten A, B, C und D mit unterschiedlichen Verhältnissen von Gewindeaußendurchmesser D_{A} zu Gewindekerndurchmesser D_{K} der inhomogenen Knochenfestigkeit eines Wirbels Rechnung und liefert so eine verbesserte Verankerung im Wirbel 20.

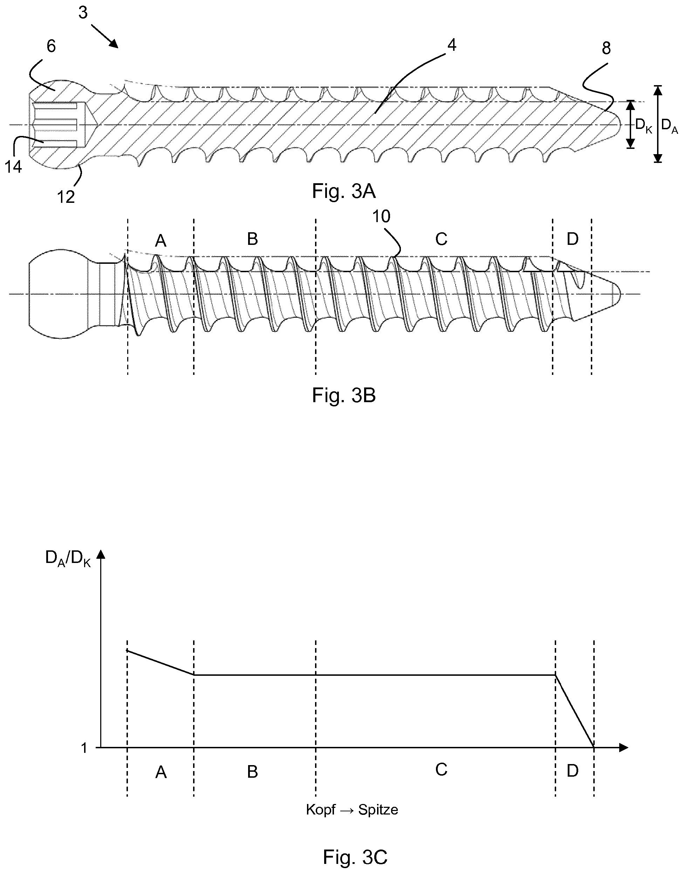

Die Fign. 3A und 3B zeigen in einer Querschnittsansicht bzw. einer Seitenansicht eine erfindungsgemäße Knochenschraube bzw. Pedikelschraube 3 gemäß einer zweiten Ausführungsform. Die Pedikelschraube 3 weist wie die Pedikelschraube 2 der ersten Ausführungsform einen länglichen Schraubenschaft 4 mit einem Außengewinde 10 auf, an dessen proximalen Ende ein Schraubenkopf 6 vorgesehen ist und der an seinem distalen Ende zu einer Schraubenspitze 8 konisch zuläuft. Im Folgenden wird deshalb nur auf die Unterschiede zur ersten Ausführungsform eingegangen.

Die Fig. 3C zeigt das (nicht maßstabsgetreue) Verhältnis von Gewindeaußendurchmesser D_{A} zu Gewindekerndurchmesser D_{K} über die Gewindelänge. Aus dem Diagramm der Fig. 3C ist zu erkennen, dass sich das Verhältnis im Übergangsbereich B nicht ändert und lediglich im Bereich des Gewindeauslaufs A zunimmt. Sowohl der Gewindekerndurchmesser D_{K} als auch Gewindeaußendurchmesser D_{A} sind in den Schraubenschaftabschnitten B und C konstant. Im Bereich des Gewindeauslaufs A bleibt der Gewindekerndurchmesser D_{K} weiterhin konstant, während der Gewindeaußendurchmesser D_{A} zum Schraubenkopf 6 hin, insbesondere stetig, zunimmt. Deshalb nimmt das Verhältnis von Gewindeaußendurchmesser D_{A} zu Gewindekerndurchmesser D_{K} zum Schraubenkopf 6 hin zu. Somit liefert auch die Pedikelschraube 3 gemäß der zweiten Ausführungsform die gleiche gute Verankerung in dem kortikalen Knochen 34 im Bereich der Eintrittsstelle 38.

Während bei der zweiten Ausführungsform im Bereich des Gewindeauslaufs der Gewindekerndurchmesser D_{K} konstant bleibt und der Gewindeaußendurchmesser D_{A} größer wird, können gemäß einer anderen (nicht dargestellten) Ausführungsform Gewindeaußendurchmesser D_{A} und Gewindekerndurchmesser D_{K} gleichzeitig im Bereich des Gewindeauslaufs A zunehmen. Mit anderen Worten reichen der konische Kerndurchmesser D_{K} und der konischen Außendurchmesser D_{A} gleichzeitig in den zylindrischen Durchmesserabschnitt B rein. In diesem Fall nimmt der Gewindeaußendurchmesser D_{A} stärker als der Gewindekerndurchmesser D_{K} zu, so dass sich auch in diesem Fall das Verhältnis von Gewindeaußendurchmesser D_{A} zu Gewindekerndurchmesser D_{K} zum Schraubenkopf 6 vergrößert, eine gute Verankerung in dem kortikalen Knochen 34 im Bereich der Eintrittsstelle 38 zu bewirken. Somit hat das Verhältnis von Gewindeaußendurchmesser D_{A} zu Gewindekerndurchmesser D_{K} bei dieser nicht in den Figuren dargestellte Ausführungsform qualitativ einen ähnlichen Verlauf wie die zweite Ausführungsform (s. Fig. 3C).

Die Fign. 4, 5, 6 und 7 zeigen zum Vergleich die Gewindeverläufe von den eingangs beschriebenen herkömmlichen Pedikelschrauben.

Die Fign. 4A bis 4C zeigen eine Pedikelschraube 40 und den entsprechenden Verlauf des Verhältnisses von Gewindeaußendurchmesser D_{A} zu Gewindekerndurchmesser D_{K} bei einem zylindrischen und über die gesamte Schaftlänge gleich bleibenden Gewindedurchmessers. Deshalb ändert sich auch, wie es aus der Fig. 4C hervorgeht, nicht das Verhältnis von Gewindeaußendurchmesser D_{A} zu Gewindekerndurchmesser D_{K}. Eine solche Pedikelschraube 40 wird somit nicht den unterschiedlichen biomechanischen Eigenschaften des Wirbels 20 gerecht.

Die Fign. 5A bis 5C zeigen entsprechende Darstellungen für eine Pedikelschraube 50, die über die gesamte Schaftlänge einen konstanten Gewindeaußendurchmesser D_{A} aufweist, wohingegen der Gewindekern zwei zylindrische Abschnitte A und C und einen dazwischen liegenden konischen Abschnitt B aufweist. Aus dem Diagramm der Fig. 5C ist zu erkennen, dass das Verhältnis von Gewindeaußendurchmesser zu Gewindekerndurchmesser zum Schraubenkopf hin eher abnimmt, was mit einer geringeren Gewindetiefe einhergeht.

Die in den Fign. 6A bis 6C dargestellte Pedikelschraube 60 unterscheidet sich von der Pedikelschraube 50 gemäß Fign. 5A bis 5C darin, dass diese im Bereich des Gewindeauslaufs A einen weiteren konischen Gewindekerndurchmesserabschnitt aufweist, in dem sich der Gewindekerndurchmesser D_{K} bei gleich bleibendem Gewindeaußendurchmesser D_{A} zum Schraubenkopf hin vergrößert, wodurch das Verhältnis von Gewindeaußendurchmesser D_{A} zu Gewindekerndurchmesser D_{K} zum Schraubenkopf hin abnimmt. Aus den Figuren ist ferner erkennbar, dass die Schraubenflanken im Bereich des Gewindeauslaufs A breiter und stumpfer werden, was zu den eingangs geschilderten Nachteilen führt.

In den Fign. 7A bis 7C ist eine Pedikelschraube 70 gezeigt, welche ein so genanntes teilkonisches Gewinde aufweist, bei dem der Kerndurchmesser D_{K} über die Schaftlänge zum Schraubenkopf hin stetig größer wird, während der Gewindeaußendurchmesser D_{A} konstant bleibt. Auch diese Pedikelschraube ist nicht in der Lage, sich im Bereich der Eintrittsstelle 38 in das kortikale Knochenmaterial 34 einzuschneiden und sich darin zu verankern.

Die erfindungsgemäße Knochenschraube wurde anhand einer Pedikelschraube 2 dargestellt, ist aber nicht auf eine solche beschränkt. Durch die Vergrößerung des Verhältnisses von Gewindeaußendurchmesser D_{A} zu Gewindekerndurchmesser D_{K} im Bereich des Gewindeauslaufs A und der damit verbundenen Vertiefung des Gewindes und besseren Verankerung in der kortikalen Hülle 34 eines Knochens kann die erfindungsgemäße Gewindegestaltung auch bei zu anderen Zwecken verwendeten Knochenschrauben genutzt werden.

## Patentansprüche

1. Pedikelschraube (2; 3), mit einem mit einem Außengewinde (10) versehenen Schraubenschaft (4) zum Verankern der Pedikelschraube (2; 3) in einem Knochen (20) und einen am proximalen Ende des Schraubenschafts (4) vorgesehenen Schraubenkopf (6),
**dadurch gekennzeichnet, dass**
in einem proximalen Schraubenschaftabschnitt (A), im Bereich des Gewindeauslaufs das Verhältnis von Gewindeaußendurchmesser (D_{A}) zu Gewindekerndurchmesser (D_{K}) zum Schraubenkopf (6) hin zunimmt und
das Verhältnis von Gewindeaußendurchmesser (D_{A}) zu Gewindekerndurchmesser (D_{K}) in einem Übergangsbereich (B) von einem medialen Schraubenschaftabschnitt (C) zu dem proximalen Schraubenschaftabschnitt (A) zunächst abnimmt, bevor es in dem proximalen Schraubenschaftabschnitt (A) zunimmt.

2. Pedikelschraube (2; 3) nach Anspruch 1, **dadurch gekennzeichnet, dass** in dem proximalen Schraubenschaftabschnitt (A) sowohl der Gewindeaußendurchmesser (D_{A}) als auch der Gewindekerndurchmesser (D_{K}) zunimmt, wobei der Gewindeaußendurchmesser (D_{A}) verhältnismäßig stärker zunimmt.

3. Pedikelschraube (2; 3) nach Anspruch 1 oder 2, **dadurch gekennzeichnet, dass** in dem proximalen Schraubenschaftabschnitt (A) sowohl der Gewindeaußendurchmesser (D_{A}) als auch der Gewindekerndurchmesser (D_{K}) linear oder exponentiell zunehmen.

4. Pedikelschraube (2; 3) nach Anspruch 1 oder 2, **dadurch gekennzeichnet, dass** in dem proximalen Schraubenschaftabschnitt (A) der Gewindeaußendurchmesser (D_{A}) exponentiell zunimmt und der Gewindekerndurchmesser (D_{K}) linear zunimmt.

5. Pedikelschraube (3) nach Anspruch 1, **dadurch gekennzeichnet, dass** in dem proximalen Schraubenschaftabschnitt (A) der Gewindekerndurchmesser (D_{K}) konstant ist und der Gewindeaußendurchmesser (D_{A}) zum Schraubenkopf hin zunimmt.

6. Pedikelschraube (2; 3) nach einem der Ansprüche 1 bis 5, **dadurch gekennzeichnet, dass** in dem medialen Schraubenschaftabschnitt (C) der Gewindeaußendurchmesser (D_{A}) und der Gewindekerndurchmesser (D_{K}) konstant bleiben.

7. Pedikelschraube (2) nach Anspruch 6, **dadurch gekennzeichnet, dass** in einem Übergangsbereich (B) vom medialen Schraubenschaftabschnitt (C) zum proximalen Schraubenschaftabschnitt (A) hin der Gewindekerndurchmesser (D_{K}) zunimmt und der Gewindeaußendurchmesser (D_{A}) konstant bleibt.

8. Pedikelschraube (2; 3) nach einem der Ansprüche 1 bis 7, **dadurch gekennzeichnet, dass** der Schraubenkopf (6) und der Schraubenschaft (4) separat ausgebildet und miteinander lösbar verbindbar sind.

9. Pedikelschraube (2; 3) nach einem der Ansprüche 1 bis 8, **dadurch gekennzeichnet, dass** mit dem Schraubenkopf (6) eine Tulpe zur Fixierung eines Längsträgers eines Wirbel-Stabilisierungssystems koppelbar ist.

## Claims

1. A pedicle screw (2; 3), comprising a screw shaft (4) provided with an external thread (10) and serving for anchoring the pedicle screw (2; 3) in a bone (20), and a screw head (6) provided on the proximal end of the screw shaft (4),
**characterized in that**
the ratio between the outer thread diameter (D_{A}) and thread core diameter (D_{K}) increases toward the screw head (6) in a proximal screw shaft portion (A) in the area of the thread's run-out, and
the ratio between the outer thread diameter (D_{A}) and the thread core diameter (D_{K}) initially decreases in a transition area (B) from a middle screw shaft portion (C) to the proximal screw shaft portion (A) before it increases in the proximal screw shaft portion (A).

2. The pedicle screw (2; 3) according to claim 1, **characterized in that** both the outer thread diameter (D_{A}) and the thread core diameter (D_{K}) increase in the proximal screw shaft portion (A), with the outer thread diameter (D_{A}) increasing to a proportionally higher degree.

3. The pedicle screw (2; 3) according to claim 1 or 2, **characterized in that** both the outer thread diameter (D_{A}) and the thread core diameter (D_{K}) increase in a linear or exponential manner in the proximal screw shaft portion (A).

4. The pedicle screw (2; 3) according to claim 1 or 2, **characterized in that** the outer thread diameter (D_{A}) increases exponentially and the thread core diameter (D_{K}) increases linearly in the proximal screw shaft portion (A).

5. The pedicle screw (3) according to claim 1, **characterized in that** the thread core diameter (D_{K}) is constant and the outer thread diameter (D_{A}) increases toward the screw head (6) in the proximal screw shaft portion (A).

6. The pedicle screw (2; 3) according to any one of claims 1 to 5, **characterized in that** the outer thread diameter (D_{A}) and the thread core diameter (D_{K}) remain constant in the middle screw shaft portion (C).

7. The pedicle screw (2) according to claim 6, **characterized in that** the thread core diameter (D_{K}) increases and the outer thread diameter (D_{A}) remains constant in the transition area (B) from the middle screw shaft portion (C) to the proximal screw shaft portion (A).

8. The pedicle screw (2; 3) according to any one of claims 1 to 7, **characterized in that** the screw head (6) and the screw shaft (4) are formed as separate parts and can be detachably connected to each other.

9. The pedicle screw (2; 3) according to any one of claims 1 to 8, **characterized in that** the screw head (6) allows to be coupled to a tulip for the fixation of a longitudinal member of a vertebral stabilization system.

## Revendications

1. Vis pédiculaire (2 ; 3) avec une tige de vis (4) dotée d'un filetage extérieur (10) pour ancrer la vis pédiculaire (2 ; 3) dans un os (20) et une tête de vis (6) prévue à l'extrémité proximale de la tige de vis (4),
**caractérisée en ce que**
dans une section de tige de vis (A) proximale, dans la zone de la sortie de filetage, le rapport du diamètre extérieur de filetage (D_{A}) au diamètre de cœur de filetage (D_{K}) augmente en allant vers la tête de vis (6) et
le rapport de diamètre extérieur de filetage (D_{A}) au diamètre de cœur de filetage (D_{K}) diminue d'abord dans une zone de transition (B) d'une section de tige de vis (C) médiane vers la section de tige de vis (A) proximale, avant d'augmenter dans la section de tige de vis (A) proximale.

2. Vis pédiculaire (2 ; 3) selon la revendication 1, **caractérisée en ce que**, dans la section de tige de vis (A) proximale, tant le diamètre extérieur de filetage (D_{A}) que le diamètre de cœur de filetage (D_{K}) augmente, dans laquelle le diamètre extérieur de filetage (D_{A}) augmente proportionnellement plus rapidement.

3. Vis pédiculaire (2 ; 3) selon la revendication 1 ou 2, **caractérisée en ce que**, dans la section de tige de vis (A) proximale, tant le diamètre extérieur de filetage (D_{A}) que le diamètre de cœur de filetage (D_{K}) augmentent de façon linéaire ou exponentielle.

4. Vis pédiculaire (2 ; 3) selon la revendication 1 ou 2, **caractérisée en ce que**, dans la section de tige de vis (A) proximale, le diamètre extérieur de filetage (D_{A}) augmente de façon exponentielle et le diamètre de cœur de filetage (D_{K}) augmente de façon linéaire.

5. Vis pédiculaire (3) selon la revendication 1, **caractérisée en ce que**, dans la section de tige de vis (A) proximale, le diamètre de cœur de filetage (D_{K}) est constant et le diamètre extérieur de filetage (D_{A}) augmente en allant vers la tête de vis.

6. Vis pédiculaire (2 ; 3) selon l'une des revendications 1 à 5, **caractérisée en ce que**, dans la section de tige de vis (C) médiane, le diamètre extérieur de filetage (D_{A}) et le diamètre de cœur de filetage (D_{K}) demeurent constants.

7. Vis pédiculaire (2) selon la revendication 6, **caractérisée en ce que**, dans une zone de transition (B) de la section de tige de vis (C) médiane en allant vers la section de tige de vis (A) proximale, le diamètre de cœur de filetage (D_{K}) augmente et le diamètre extérieur de filetage (D_{A}) demeure constant.

8. Vis pédiculaire (2 ; 3) selon l'une des revendications 1 à 7, **caractérisée en ce que** la tête de vis (6) et la tige de vis (4) sont conçues séparément et peuvent être reliées ensemble de façon amovible.

9. Vis pédiculaire (2 ; 3) selon l'une des revendications 1 à 8, **caractérisée en ce qu'**une tulipe peut être couplée à la tête de vis (6) pour fixer un support longitudinal d'un système de stabilisation de vertèbre.
